(19) Europäisches Patentamt
European Patent Office
Office européen des brevets

(11) Publication number: **0 373 658 A2**

(12) **EUROPEAN PATENT APPLICATION**

(21) Application number: **89123181.3**

(51) Int. Cl.[5]: **A61K 31/47**

(22) Date of filing: **14.12.89**

Claims for the following Contracting States: ES + GR.

The title of the invention has been amended (Guidelines for Examination in the EPO, A-III, 7.3).

(30) Priority: **16.12.88 GB 8829330**

(43) Date of publication of application: **20.06.90 Bulletin 90/25**

(84) Designated Contracting States: **AT BE CH DE ES FR GB GR IT LI LU NL SE**

(71) Applicant: **SANDOZ AG**
**Lichtstrasse 35**
**CH-4002 Basel(CH)**
(84) **BE CH ES FR GB GR IT LI LU NL SE**

Applicant: **SANDOZ-PATENT-GMBH**
**Humboldtstrasse 3**
**D-7850 Lörrach(DE)**
(84) **DE**

Applicant: **SANDOZ-ERFINDUNGEN Verwaltungsgesellschaft m.b.H.**
**Brunner Strasse 59**
**A-1235 Wien(AT)**
(84) **AT**

(72) Inventor: **Brownell, Judith**
**Sierentzerstrasse 9**
**CH-4055 Basel(CH)**

(54) **Use of quinoline derivatives in treating cancer.**

(57) Low molecular weight quinoline derivatives, in free form or in pharmaceutically acceptable salt form are useful in reducing the spread or growth of secondary cancer cells associated with treatment of a primar cancer tumor, e.g. perioperative treatment of primary operable cancer tumors, and in treating meningiomas.

EP 0 373 658 A2

## CANCER TREATMENT

The present invention relates to a new use, in particular a new pharmaceutical use, for the compound group comprising low molecular weight quinoline derivatives, their physiologically-hydrolysable and -acceptable esters and their pharmaceutically acceptable salts, said compound group being referred to hereinafter collectively as COMPOUNDS OF THE INVENTION.

The COMPOUNDS OF THE INVENTION may comprise a nucleus of e.g. up to fused 4 rings including the quinoline moiety. The nucleus may bear substituents, e.g. side-chains. The compounds are low molecular weight in the sense that each side-chain does not contain any fused rings.

Examples of low molecular weight quinoline derivatives are 6-and/or 7-oxy-trans-1,2,3,4,4a,5,10,10a-octahydro-benzo[g]quinolines in which the 3-position is substituted by an optionally amidated carboxy group, an optionally etherified hydroxymethyl group, a cyanomethyl group, an alkyl- or aryl-thiomethyl group or a sulfamoylamino or carbamoylamino group, particularly benzoquinolines of formula I

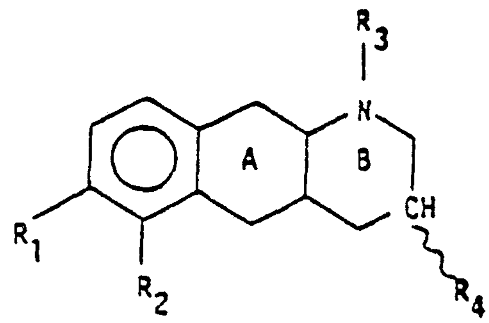

(I)

wherein the rings A and B are trans-fused and wherein

$R_1$ and $R_2$ are each independently hydrogen, hydroxy or methoxy, with the proviso that $R_1$ and $R_2$ may not both be hydrogen,

$R_3$ is hydrogen or $C_{1-4}$alkyl,

$R_4$ is $-COOH$, $-CH_2OR_5$, $-CH_2CN$, $-CON(R_6)R_7$, $-CH_2SR_8$, $-NHSO_2N(R_9)R_{10}$ or $-NHCON(R_9)R_{10}$,

$R_5$ is hydrogen or $C_{1-3}$alkyl,

$R_7$ is hydrogen, $C_{1-3}$alkyl, phenyl or pyridyl, said phenyl or pyridyl being optionally substituted by halogen, methyl or methoxy or

$R_6$ and $R_7$ together are $-(CH_2)_4$, $-(CH_2)_5-$ or $-(CH_2)_2-O-(CH_2)_2-$,

$R_8$ is $C_{1-4}$alkyl or pyridyl, said pyridyl being optionally substituted by halogen, methyl or methoxy, and

$R_9$ and $R_{10}$ are each independently hydrogen or $C_{1-3}$alkyl or together are $-(CH_2)_4-$ or $-(CH_2)_5-$,

as well as the physiologically-hydrolysable and -acceptable esters and pharmaceutically acceptable salts thereof.

By the term "physiologically-hydrolysable and -acceptable esters" is meant esters with acids or alcohols which are hydrolysable under physiological conditions to yield acids and alcohols which are themselves physiologically acceptable, i.e. which are non-toxic at the desired dosage levels. Such esters may be obtained by acylation of said benzo[g]quinolines bearing one or more hydroxy residues, e.g. hydroxy and/or hydroxymethyl groups at the 3-, 6-and/or 7-position and/or esterification of said benzo[g]-quinolines bearing an acidic residue, e.g. a carboxy group at the 3-position. Such esters include esters with mono- and di-carboxylic acids in particular carboxylic acids having 2 to 5 carbon atoms, as well as esters with aliphatic alcohols having 1 to 4 carbon atoms.

These compounds are known from e.g. European Patent Publication 77754 (A2), the contents of which including all the examples are incorporated herein by reference.

The preferred compound thereof is

N,N-diethyl-N′-[(3α,4aα,10aβ)-1,2,3,4,4a,5,10,10a-octahydro-6-hydroxy-1-propyl-3-benzo[g]-quinolinyl] sulfamide, also known as CV, (hereinafter compound A) preferably used in salt from, particularly as the hydrochloride.

Further examples of low molecular weight quinoline derivatives are e.g. 1-ethyl-3-(3-dimethylaminopropyl)-3-(1-allyl-6-methoxy-1,2,3,4,4aα,5,10,10aβ-octahydrobenzo[g]quinoline-3β-carbonyl)-urea, and 1-ethyl-3-(3-dimethylaminopropyl)-3-(1-propyl-6-methoxy-1,2,3,4,4aα,5,10,10aβ-octahydrobenzo-[g]quinoline-3β-carbonyl)-urea.

A further preferred group of COMPOUNDS OF THE INVENTION is comprised by low molecular weight ergot derivatives, i.e. compounds which do not have a peptide moiety in the 8 position, i.e. not ergotpeptides. They may have for example an amino group, e.g. an acylamino, ureidio or sulphamino moiety or thiomethyl moiety in the 8 position which may be substituted by for example one or if desired two $(C_{1-4})$alkyl groups. Conveniently these have a single bond in the 9,10 position of the ergoline nucleus.

Preferred compounds are 8α-sulphamoylamino ergolines, for example compounds of the formula Ia

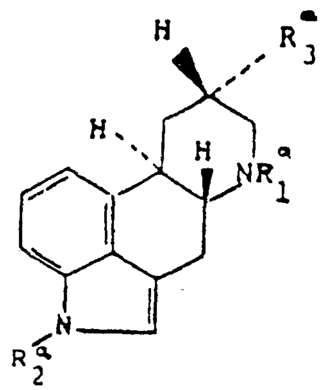

(Ia)

wherein $R_1^a$ inter alia is $(C_{1-4})$alkyl,
$R_2^a$ inter alia is H or $(C_{1-4})$alkyl,
$R_3^a$ inter alia is $-NHSO_2N[(C_{1-4})alkyl]_2$.

Individual compounds suitable for use in accordance with the invention are:

a) 1,6-dimethyl-8α-(N,N-dimethylsulphamoylamino)-ergoline-I-(also known as Mesulergine hereinafter compound B);

b) 6-n-propyl-8α-(N,N-diethylsulphamoylamino)-ergoline-I-(N,N-diethyl-N′-(6-propylergolin-8α-yl)-sulfamide) preferably used as the hydrochloride, known as CQP, (hereinafter compound C);

c) N,N-diethyl-N′-[(8α)-1-ethyl-6-methyl-ergolin-8-yl]-sulfamide preferably used as the hydrochloride, (hereinafter compound D).

Other examples of individual compounds include:

i) 3-(9,10-didehydro-6-methyl-ergolin-8α-yl)-1,1-diethyl-urea (also known as Lisuride preferably used as the hydrogen maleate);

ii) 6-n-propyl-8α-methylmercaptomethyl-ergoline-I (also known as Pergolide preferably used as the mesylate);

iii) Transhydrolisuride also known as terguride having the chemical name 3-(6-methyl-ergolin-8α-yl)-1,1-diethyl-urea, published e.g. in DOS 3135305 and 3124714.

iv) 6-n-propyldihydro-lisuride also known as proterguride having the chemical name 3-(6-n-propyl-ergolin-8α-yl)-1,1-diethyl-urea.

v) 6- and 2-substituted, e.g. 6-n-propyl and/or 2-methyl or bromo derivatives of terguride, lisuride and proterguride e.g. as published in European Patent Publication No. 21206 (A.1) and 160842 (A.1) the contents of which especially the examples and pharmacological data thereof, are incorporated herein by reference. Examples include 2-bromerguride, also known as 2-bromolisuride, preferably used in the form of the hydrochloride.

vi) Metergoline, also known as (+)-N-(carboxy)-1-methyl-9,10-dihydrolysergamine benzyl ester.

vii) Dosergoside, also known as N-(1S,2R,3E)-2-hydroxy-1-(hydroxymethyl)-3-heptadecanyl)-6-methylergoline-8-beta-carboxamide.

viii) FCE-21336 also known as 1-ethyl-3-(3′-dimethylaminopropyl)-3-(6-alkyl-ergoline-8′-beta-carbonyl)-urea preferably used as the diphosphate.

ix) GYKI-32887 also known as 6-methyl-8-(N-mesyl-N-2-azidoethyl)-ergolene preferably used as the bimaleate, e.g. as disclosed in USP 4,299,836.

Another group of preferred compounds are for example:

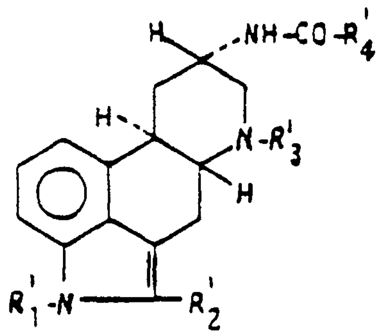

(I′)

wherein
$R_1'$ is hydrogen or $C_{1-4}$alkyl,
$R_2'$ is hydrogen, chlorine, bromine or methyl,
$R_3'$ is $C_{1-5}$alkyl or $C_{3-5}$alkenyl in which the double bond is not at the carbon atom adjacent to the nitrogen atom, and
$R_4'$ is $C_{3-7}$alkyl; $C_{3-7}$cycloalkyl; adamantyl; phenyl; phenyl substituted by one or more members selected from the group consisting of $C_{1-3}$alkyl, $C_{1-3}$alkoxy, $C_{1-3}$alkylthio, trifluoromethyl, hydroxy, nitro, amino and mono- and di($C_{1-3}$alkyl)-amino; or phenyl bearing a condensed non-aromatic, heterocyclic ring having 5- or 6-ring members including 1 or 2 hetero atoms selected from the group consisting of oxygen and sulphur, published in GB 2,152,507 A, the contents of which especially the examples and pharmacological data thereof are incorporated herein by reference, e.g. (5R,8S,10R)-2,6-dimethyl-8α-pivaloylamino-ergoline (hereinafter compound E) preferably used as the hydrochloride, and the 2-chloro derivative, (5R,8S,10R)-2-chloro-6-methyl-8α-pivaloylamino-ergoline.

Other examples include compounds of formula II″

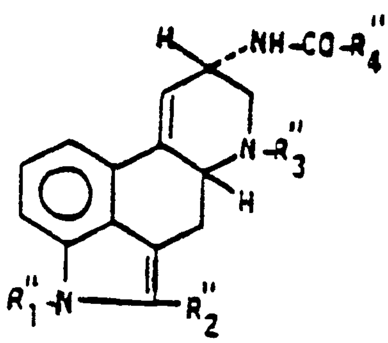

(II″)

wherein
$R_1''$ is hydrogen or $C_{1-4}$alkyl,
$R_2''$ is hydrogen, chlorine, bromine or methyl,
$R_3''$ is $C_{1-5}$alkyl or $C_{3-5}$alkenyl in which the double bond is not at the carbon atom adjacent to the nitrogen atom, and
$R_4$ is $C_{1-7}$alkyl; $C_{3-7}$cycloalkyl; adamantyl; phenyl; phenyl substituted by one or more members selected from the group consisting of $C_{1-3}$alkyl, $C_{1-3}$alkoxy, $C_{1-3}$alkylthio, trifluoromethyl, hydroxy, nitro, amino and mono- and di-($C_{1-3}$alkyl)-amino; or phenyl fused with a non-aromatic, heterocyclic ring having 5- or 6-ring members including 1 or 2 hetero atoms selected from the group consisting of oxygen and/or sulphur,
with the proviso that when $R_2''$ is hydrogen, neither $R_3''$ nor $R_4''$ is methyl, e.g. the compounds wherein $R_1''$ = H $R_2''$ = Br or especially $CH_3''$, $R_3''$ = $CH_3$ and $R_4''$ = tert butyl, filed off German Application P 3447383.1 filed 24 Dec 1984 as English application No. 8531420, now published as G.B. Application No. 2169291 A and also in other countries, the contents of which including all the examples thereof are incorporated herein by reference.

These compounds may be used for example in free base form or in pharmaceutically acceptable acid addition salt form, e.g. the hydrochloride, maleate or mesylate.

Many of the above mentioned compounds are published as prolactin secretion inhibitors, some of which

are extremely potent and have long lasting activity. The potency of the compounds as prolactin secretion inhibitors can be determined in standard tests, e.g. by lowering basal serum prolactin levels in the male rat.

Results with representative compounds include:-

| Compound | $ID_{50}$ mg/kg after 4 hours |
|---|---|
| A | 0.03 |
| B | 0.003 |
| C | 0.001 |

A group of compounds comprises compounds which are $D_2$ agonists without any antagonistic activity. Another group of compounds includes those compounds which are serotonin agonists, e.g. as indicated in standard in vitro binding tests and by activation of 5-HT sensitive adenylate cyclase of the liver fluke according to the method of R.Markstein, Neurochemical Effects of Some Ergot Derivatives: A Basis for their Antiparkinson Actions. J. Neural transm 51:39, (1981).

Examples of such compounds include compound C and pergolide. Compound C, pergolide and 5-HT agonists have efficacies of 90, 80, and 100 respectively and $pD_2$ [10g $EC_{50}$] of 6.9, 6.9 and 7.7 respectively in this test on adenylate cyclase stimulated by serotonin.

As indicated below surprisingly the potency of a COMPOUND OF THE INVENTION as a prolactin secretion inhibitor may serve as a guide to the potency in the method of the present invention, but, at least before the date of this invention, there is no evidence of a direct correlation between their ability to lower prolactin levels and their effect in the method of the present invention.

In accordance with the present invention it has now surprisingly been found that COMPOUNDS OF THE INVENTION have a beneficial effect in the treatment of cancers, particularly on primary cancers and on meningiomas.

Treatment of cancer is often directed to the primary tumor and metastases, whether clinically apparent or microscopic. Surgery to remove a primary tumor is one of the most effective forms of cancer therapy. For example, cancers of the oral cavity, larynx, lung, colon, prostate, kidney, testis, bladder, ovary, endometrium, cervix and breast may be curable by surgery.

However, in cases of malignant cancer more metastatic tumor deposits, or metastatic nodules, may have already been created in parts of the body remote from the primary tumor, prior to surgery due to the shedding of tumor cell emboli into the blood circulation from the primary tumor.

There is evidence that metastasis is not a random event and that the primary tumor exhibits an inhibitory effect on the growth of metastatic nodules. Removal of a primary tumor may result in an explosion of metastases within a short period of time.

In accordance with the particular findings of the present invention, the present invention provides, in a first aspect:

1. A method of reducing the spread or growth of secondary cancer cells associated with treatment of a primary cancer tumor in a subject which comprises administering an effective amount of a COMPOUND OF THE INVENTION to a subject in need of such treatment.

2. A method of preventing metastases of cancer cells throughout a subject associated with treatment to remove a primary cancer tumor from a subject which comprises administering an effective amount of a COMPOUND OF THE INVENTION to a subject in need of such treatment.

Examples of primary cancer tumors to which the above methods are applicable include for example meningiomas or tumors associated with cancer of the cervix, breast, bladder, colon, prostate, larynx, endometrium, ovary, oral cavity, kidney, testis, e.g. non-seminomatous, and lung e.g. non-small cell, especially breast cancers.

In a series of specific or alternative embodiments, the present invention also provides:

3. A method of treating primary operable cancer tumors in a subject which comprises administering perioperatively an effective amount of a COMPOUND OF THE INVENTION to a subject in need of such treatment.

COMPOUNDS OF THE INVENTION are particularly useful in the treatment of meningiomas, especially primary breast cancers.

4. A method of treating meningiomas in a subject which comprises administering an effective amount of a COMPOUND OF THE INVENTION to a subject in need of such treatment.

As alternatives to the above the present invention also provides:

5. A COMPOUND OF THE INVENTION for use in any method as defined under 1 to 4 above;

6. A COMPOUND OF THE INVENTION for use in the preparation of a pharmaceutical composition for use in any method as defined under 1 to 4 above;

7. A composition for use in any method as defined under 1 to 4 above comprising a COMPOUND OF THE INVENTION together with one or more pharmaceutically acceptable diluents or cariers therefor.

Utility of COMPOUNDS OF THE INVENTION in treating diseases and conditions as hereinabove specified, may be demonstrated in standard pharmacological test methods as well as in clinic, for example in accordance with the methods hereinbefore described.

## A. CLINICAL TRIAL I: REDUCTION IN THE METASTATIC RATE OF TUMOR GROWTH

The trial is effected on a randomized double blind basis.

A group of women, e.g. 30 to 40, of ca. 50 - 60 years old, having breast lumps (e.g. ca. 3 cm in diameter) admitted to hospital for excision biopsy under general anaesthesia are selected. Women with both benign or malignant cancers are included, e.g. having a Ductal grade II or III.

The women are given an oral daily dose of a COMPOUND OF THE INVENTION for 4 days before definite surgery to remove the primary tumor (e.g. mastectomy) and for 5 days thereafter, or are given corresponding placebo. The sub-groups treated by the COMPOUND OF THE INVENTION and by placebo have similar mean ages, menstrual status, and tumor grades.

DNA flow cytometry is effected on cell suspensions prepared from formalin-fixed paraffin-embedded blocks of the malignant primary tumors. From representative blocks, 50 microns thick sections are cut, dewaxed in xylene and rehydrated through a series of alcohols with distilled water. The sections are desaggregated by treatment for 30 minutes in a 5 mg/ml solution of pepsin at pH 1.5. The suspension is centrifuged at 850 g for 3 minutes and resuspended in 5 ml of a 1 µg/ml solution of DAPI (4′,6-diaminido-2-phenylindole hydrochloride. The suspension is passed through a 25 gauge needle and filtered through a 35 micron pore nylon gauze.

10,000 - 15,000 cells are analysed for the S-phase fraction (i.e. the fraction containing cells in the S-phase), according to the method of H.Baisch et al. Radiat.Environ.Biophys. (1975), 12, 31-39.

Subjects receive COMPOUNDS OF THE INVENTION, e.g. compound A, at dosages of from 50 to 200 µg/g/p.o./day, preferably in a single dose.

For the placebo sub-group the median S-phase fraction is about 10 per cent of the total.

For the sub-group treated with the COMPOUND OF THE INVENTION there is a significant decrease in the S-phase fraction. This reduction indicates a reduction of the mitotic rate not only of the primary tumor but of remote micrometastatic cell clumps.

## B. CLINICAL TRIAL II: ANTIPROLIFERATIVE EFFECT

24 to 36 women with operable breast cancer, aged less than 70 years, in whom a positive biopsy cut has been obtained at the first clinic visit are selected. The patients are not be taking any chronic medication which is known to influence serum prolactin levels. The trial is effected on a randomized double blind basis. All patients are to have baseline evaluations before beginning treatment.

The women are given an oral dose of a COMPOUND OF THE INVENTION or placebo for 20 days, starting on day 0. They are operated on day 10 with either breast conservation or modified radical mastectomy. Prolactin levels in plasma are measured on days 0, 6, 10, 13 and 20. Histological evaluations are performed on days 1 and 10.

On administration of a COMPOUND OF THE INVENTION at a dosage of from 50 to 200 ug/day/p.o., e.g. compound A at an escalating dose schedule of 25 µg/day for days 0 to 3, 50 µg/day for days 4 to 6 and 75 µg/day until day 20 once daily at bedtime, satisfactory results indicative of a reduced mitotic rate are obtained.

Equivalent results may be obtained in trials performed in relation to other primary cancers employing compound A, at the same or equivalent dosage levels to those described above. Other COMPOUNDS OF THE INVENTION may be used at doses employed to inhibit prolactin secretion.

## C. INHIBITORY EFFECT ON GROWTH OF MENINGIOMA

Freshly resected meningioma tissue is cut into small pieces which are incubated for 24 hours at 37° C. The resulting cell dispersion is seeded into flasks and grown to confluence. Cells are incubated with varying doses of COMPOUNDS OF THE INVENTION and the cultures are grown for 6 to 12 days. Thereafter the number of cells in each flask is determined by a standard method, e.g. counting Zapponin released nuclei with a Coulter-Counter.

COMPOUNDS OF THE INVENTION, in particular compound A, are active in the above test method in inhibiting meningioma cell growth at a concentration of 0.05 to 10 µmol/l.

For the above-mentioned uses the dose will, of course, vary depending on the mode of administration, the particular COMPOUND OF THE INVENTION employed, the particular condition to be treated and the therapy desired. In general, however, satisfactory results are obtained when administered at dosage sufficient to produce a long lasting and continuous prolactin secretion inhibition, e.g. to under 2 nanograms prolactin per ml blood plasma or at doses up to about 2 times higher in order to suppress any sudden prolactin surges. For example, an indicated daily dose is from about 0.001 to 10 mg (e.g. 0.01 to 10 mg) p.o. for the COMPOUNDS OF THE INVENTION, conveniently administered for example in divided doses up to 4 times a day or preferably in sustained release form. Suitable unit dosage forms for oral administration contain for example from about 0.2 µg to about 10 mg of the compounds.

The preferred compound is compound A. This produces in humans a long-lasting prolactin secretion inhibiting effect at 0.01 mg to 0.2 mg p.o. per day.

The invention is especially interesting when the primary tumors are removed by surgery. Radiotherapy or chemotherapy may alternatively or additionally be employed.

The COMPOUNDS OF THE INVENTION may be administered concomitantly with other therapy, e.g. radiotherapy, or anti-neoplastic chemotherapy, such as alkylating agents, e.g. cyclophosphamide, anti-metabolites, plant alkaloids, anti-tumor antibiotics, inorganic ions such as cis-platin, biological response modifiers such as interferons, enzymes such as L-asparaginase or other endocrine therapy.

The COMPOUNDS OF THE INVENTION may be administered in free base form or in pharmaceutically acceptable acid addition salt form. Such compositions are in general known and may be produced in conventional manner, e.g. by bringing a COMPOUND OF THE INVENTION into intimate admixture with the pharmaceutically acceptable diluent or carrier and effecting formulation or presentation so as to provide for or permit convenient administration. The COMPOUNDS OF THE INVENTION may be administered by any conventional route in particular enterally, preferably orally, e.g. in the form of capsules or tablets, or if appropriate parenterally in the form of injectable solutions or suspensions.

The following is illustrative of the preparation of a solid composition in accordance with the invention.

**EXAMPLE: Production of solid compositions for oral application**

Tablets or capsules may contain the active agent in admixture with conventional pharmaceutically acceptable excipients, e.g. inert diluents, such as calcium carbonate, sodium carbonate, lactose and talc, granulating and disintegrating agents, e.g. starch and alginic acid, flavouring, colouring and sweetening agents, binding agents, e.g. starch, gelatin and acacia, and lubricating agents, magnesium stearate, stearic acid and talc.

The following example is illustrative for the preparation of capsule forms:

| INGREDIENTS | WT/DOSE |
|---|---|
| Compound A (0.1 mg base) as hydrochloride | 0.1092 mg |
| Corn starch | 175.3908 mg |
| Lactose (200 mesh) | 120.0 mg |
| Silica | 1.5 mg |
| Magnesium stearate | 3.0 mg |
| Total | 300 mg |

Analogously a capsule containing 25, 50 or 75 µg of compound A may be made.

The active ingredients are intimately admixed employing conventional galenic procedures, filled into hard gelatin capsules and the capsules sealed. Capsule weight = 97.0 mg: Total weight for filled capsule = 397.00 mg

COMPOUNDS OF THE INVENTION are well tolerated at dosages required for use in accordance with

the present invention.

Thus in 52 week toxicology studies in which compound A is given to rats in oral doses, no toxic effects are observed on administration of doses of 0.20 mg/kg/day.

Pharmaceutically acceptable salt forms exhibit the same or similar levels of tolerability/activity as the free compounds.

## Claims

1. Use of a low molecular weight quinoline derivative, in free form or in pharmaceutically acceptable salt form, in the preparation of a pharmaceutical composition for use in reducing the spread or growth of secondary cancer cells associated with treatment of a primary cancer tumor.

2. Use of a low molecular weight quinoline derivative, in free form or in pharmaceutically acceptable salt form, in the preparation of a pharmaceutical composition for use in preventing metastases of cancer cells throughout a subject associated with treatment to remove a primary cancer tumor.

3. Use of a low molecular weight quinoline derivative, in free form or in pharmaceutically acceptable salt form, in the preparation of a pharmaceutical composition for use in perioperative treatment of primary operable cancer tumors.

4. Use according to claim 3 of a low molecular weight quinoline derivative, in free form or in pharmaceutically acceptable salt form, in the preparation of a pharmaceutical composition for use in perioperative treatment of primary operable breast cancer.

5. Use of a low molecular weight quinoline derivative, in free form-or in pharmaceutically acceptable salt form, in the preparation of a pharmaceutical composition for use in treating meningiomas.

6. Use according to any one of the preceding claims, in which the quinoline derivative is a 6- and/or 7-oxy-trans-1,2,3,4,4a,5,10,10a-octahydro-benzo[g]quinolines in which the 3-position is substituted by an optionally amidated carboxy group, an optionally etherified hydroxymethyl group, a cyanomethyl group, an alkyl- or aryl-thiomethyl group or a sulfamoylamino or carbamoylamino group.

7. Use according to claim 6, in which the quinoline derivative is a compound of formula I

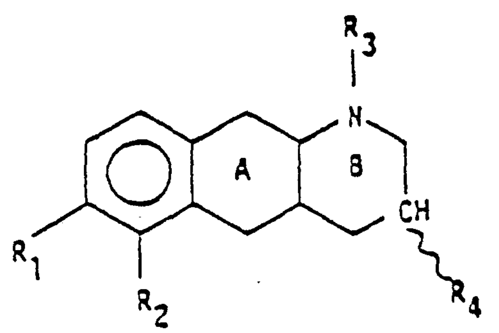

(I)

wherein the rings A and B are trans-fused and wherein

$R_1$ and $R_2$ are each independently hydrogen, hydroxy or methoxy, with the proviso that $R_1$ and $R_2$ may not both be hydrogen,

$R_3$ is hydrogen or $C_{1-4}$alkyl,

$R_4$ is $-COOH$, $-CH_2OR_5$, $-CH_2CN$, $-CON(R_6)R_7$, $-CH_2SR_8$, $-NHSO_2N(R_9)R_{10}$ or $-NHCON(R_9)R_{10}$,

$R_5$ is hydrogen or $C_{1-3}$alkyl,

$R_7$ is hydrogen, $C_{1-3}$alkyl, phenyl or pyridyl, said phenyl or pyridyl being optionally substituted by halogen, methyl or methoxy or

$R_6$ and $R_7$ together are $-(CH_2)_4$, $-(CH_2)_5$- or $-(CH_2)_2$-O-$(CH_2)_2$-,

$R_8$ is $C_{1-4}$alkyl or pyridyl, said pyridyl being optionally substituted by halogen, methyl or methoxy, and

$R_9$ and $R_{10}$ are each independently hydrogen or $C_{1-3}$alkyl or together are $-(CH_2)_4$- or $-(CH_2)_5$-,

as well as the physiologically-hydrolysable and -acceptable esters and pharmaceutically acceptable salts thereof.

8. Use according to claim 7, in which the quinoline derivative is N,N-diethyl-N′-[(3α,4aα,10aβ)-1,2,3,4,4a,5,10,10a-octahydro-6-hydroxy-1-propyl-3-benzo[g]quinolinyl] sulfamide, in free form or in pharmaceutically acceptable salt form.

9. Use according to claim 8, in which the quinoline derivative is in the form of hydrochloride.

10. Use according to any one of claim 1 to 5, in which the quinoline derivative is a compound of formula Ia

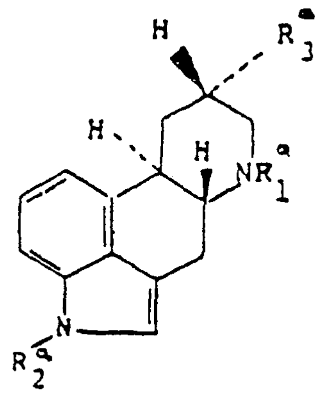

(Ia)

wherein $R_1{}^a$ inter alia is $(C_{1-4})$alkyl,
$R_2{}^a$ inter alia is H or $(C_{1-4})$alkyl,
$R_3{}^a$ inter alia is $-NHSO_2N[(C_{1-4})alkyl]_2$
a compound of formula I′

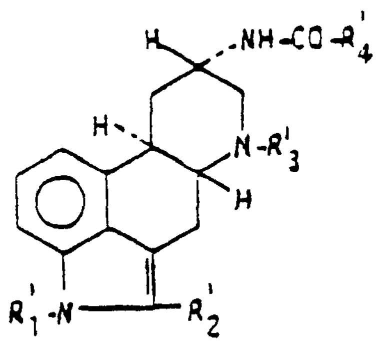

(I′)

wherein
$R_1'$ is hydrogen or $C_{1-4}$alkyl,
$R_2'$ is hydrogen, chlorine, bromine or methyl,
$R_3'$ is $C_{1-5}$alkyl or $C_{3-5}$alkenyl in which the double bond is not at the carbon atom adjacent to the nitrogen atom, and
$R_4'$ is $C_{3-7}$alkyl; $C_{3-7}$cycloalkyl; adamantyl; phenyl; phenyl substituted by one or more members selected from the group consisting of $C_{1-3}$alkyl, $C_{1-3}$alkoxy, $C_{1-3}$alkylthio, trifluoromethyl, hydroxy, nitro, amino and mono-and di-$(C_{1-3}$alkyl)-amino; or phenyl bearing a condensed non-aromatic, heterocyclic ring having 5- or 6-ring members including 1 or 2 hetero atoms selected from the group consisting of oxygen and sulphur, or a compound of formula II″

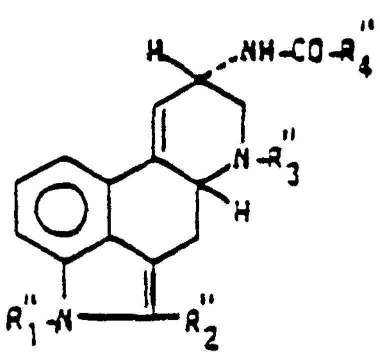

(II″)

wherein
$R_1''$ is hydrogen or $C_{1-4}$alkyl,
$R_2''$ is hydrogen, chlorine, bromine or methyl,
$R_3''$ is $C_{1-5}$alkyl or $C_{3-5}$alkenyl in which the double bond is not at the carbon atom adjacent to the nitrogen atom, and
$R_4$ is $C_{1-7}$alkyl; $C_{3-7}$cycloalkyl; adamantyl; phenyl; phenyl substituted by one or more members selected from the group consisting of $C_{1-3}$alkyl, $C_{1-3}$alkoxy, $C_{1-3}$alkylthio, trifluoromethyl, hydroxy, nitro, amino and mono- and di-($C_{1-3}$alkyl)amino; or phenyl fused with a non-aromatic, heterocyclic ring having 5- or 6-ring members including 1 or 2 hetero atoms selected from the group consisting of oxygen and/or sulphur,
with the proviso that when $R_2''$ is hydrogen, neither $R_3''$ nor $R_4''$ is methyl,
in free form or in pharmaceutically acceptable salt form.

Claims for the following Contracting States: GR,ES

1. A process for the preparation of a composition comprising a low molecular weight quinoline derivative, in free form or in pharmaceutically acceptable salt form, for use in reducing the spread or growth of secondary cancer cells associated with treatment of a primary cancer tumor, which process comprises bringing said low molecular weight quinoline derivative into intimate admixture with a pharmaceutically acceptable diluent or carrier and effecting formulation or presentation so as to provide for or permit convenient administration.

2. A process for the preparation of a composition comprising a low molecular weight quinoline derivative, in free form or in pharmaceutically acceptable salt form, for use in preventing metastases of cancer cells throughout a subject associated with treatment to remove a primary cancer tumor, which process comprises bringing said low molecular weight quinoline derivative into intimate admixture with a pharmaceutically acceptable diluent or carrier and effecting formulation or presentation so as to provide for or permit convenient administration.

3. A process for the preparation of a composition comprising a low molecular weight quinoline derivative, in free form or in pharmaceutically acceptable salt form, for use in perioperative treatment of primary operable cancer tumors, which process comprises bringing said low molecular weight quinoline derivative into intimate admixture with a pharmaceutically acceptable diluent or carrier and effecting formulation or presentation so as to provide for or permit convenient administration.

4. A process according to claim 3 for the preparation of a composition for use in perioperative treatment of primary operable breast cancer.

5. A process for the preparation of a composition comprising a low molecular weight quinoline derivative, in free form or in pharmaceutically acceptable salt form, for use in treating meningiomas, which process comprises bringing said low molecular weight quinoline derivative into intimate admixture with a pharmaceutically acceptable diluent or carrier and effecting formulation or presentation so as to provide for or permit convenient administration.

6. A process according to any one of the preceding claims, in which the quinoline derivative is a 6- and/or 7-oxy-trans-1,2,3,4,4a,5,10,10a-octahydro-benzo[g]quinolines in which the 3-position is substituted by an optionally amidated carboxy group, an optionally etherified hydroxymethyl group, a cyanomethyl group, an alkyl- or aryl-thiomethyl group or a sulfamoylamino or carbamoylamino group.

7. A process according to claim 6, in which the quinoline derivative is a compound of formula I

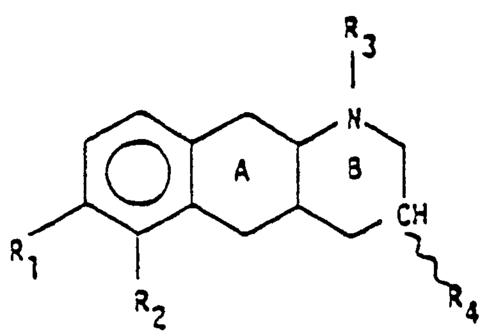
, (I)

wherein the rings A and B are trans-fused and wherein
$R_1$ and $R_2$ are each independently hydrogen, hydroxy or methoxy, with the proviso that $R_1$ and $R_2$ may not

both be hydrogen,
$R_3$ is hydrogen or $C_{1-4}$alkyl,
$R_4$ is -COOH, $-CH_2OR_5$, $-CH_2CN$, $-CON(R_6)R_7$, $-CH_2SR_8$, $-NHSO_2N(R_9)R_{10}$ or $-NHCON(R_9)R_{10}$,
$R_5$ is hydrogen or $C_{1-3}$alkyl,
$R_7$ is hydrogen, $C_{1-3}$alkyl, phenyl or pyridyl, said phenyl or pyridyl being optionally substituted by halogen, methyl or methoxy or
$R_6$ and $R_7$ together are $-(CH_2)_4$, $-(CH_2)_5-$ or $-(CH_2)_2-O-(CH_2)_2-$,
$R_8$ is $C_{1-4}$alkyl or pyridyl, said pyridyl being optionally substituted by halogen, methyl or methoxy, and
$R_9$ and $R_{10}$ are each independently hydrogen or $C_{1-3}$alkyl or together are $-(CH_2)_4-$ or $-(CH_2)_5-$,
as well as the physiologically-hydrolysable and -acceptable esters and pharmaceutically acceptable salts thereof.

8. A process according to claim 7, in which the quinoline derivative is N,N-diethyl-N′-[(3α,4aα,10aβ)-1,2,3,4,4a,5,10,10a-octahydro-6-hydroxy-1-prcpyl-3-benzo[g]quinolinyl]sulfamide, in free form or in pharmaceutically acceptable salt form.

9. A process according to claim 8, in which the quinoline derivative is in the form of hydrochloride.

10. A process according to any one of claims 1 to 5, in which the quinoline derivative is a compound of formula Ia

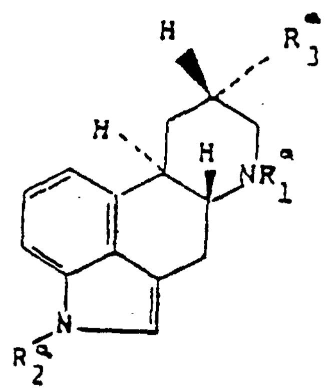

(Ia)

wherein $R_1{}^a$ inter alia is $(C_{1-4})$alkyl,
$R_2{}^a$ inter alia is H or $(C_{1-4})$alkyl,
$R_3{}^a$ inter alia is $-NHSO_2N[(C_{1-4})alkyl]_2$
a compound of formula I′

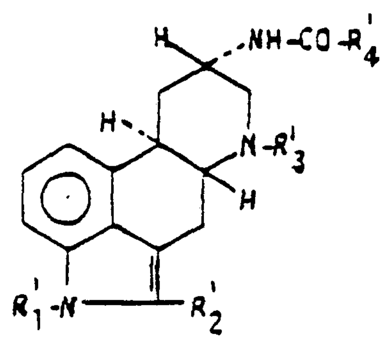

(I′)

wherein
$R_1'$ is hydrogen or $C_{1-4}$alkyl,
$R_2'$ is hydrogen, chlorine, bromine or methyl,
$R_3'$ is $C_{1-5}$alkyl or $C_{3-5}$alkenyl in which the double bond is not at the carbon atom adjacent to the nitrogen atom, and
$R_4'$ is $C_{3-7}$alkyl; $C_{3-7}$cycloalkyl; adamantyl; phenyl; phenyl substituted by one or more members selected from the group consisting of $C_{1-3}$alkyl, $C_{1-3}$alkoxy, $C_{1-3}$alkylthio, trifluoromethyl, hydroxy, nitro, amino and mono-and di-($C_{1-3}$alkyl)-amino; or phenyl bearing a condensed non-aromatic, heterocyclic ring having 5- or

6-ring members including 1 or 2 hetero atoms selected from the group consisting of oxygen and sulphur, or a compound of formula II''

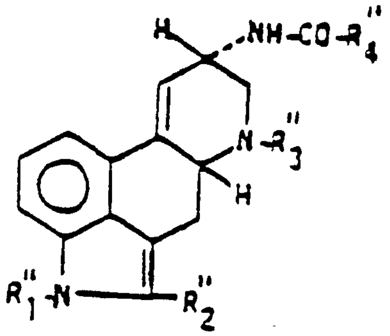

(II'')

wherein
$R_1''$ is hydrogen or $C_{1-4}$alkyl,
$R_2''$ is hydrogen, chlorine, bromine or methyl,
$R_3''$ is $C_{1-5}$alkyl or $C_{3-5}$alkenyl in which the double bond is not at the carbon atom adjacent to the nitrogen atom, and
$R_4$ is $C_{1-7}$alkyl; $C_{3-7}$cycloalkyl; adamantyl; phenyl; phenyl substituted by one or more members selected from the group consisting of $C_{1-3}$alkyl, $C_{1-3}$alkoxy, $C_{1-3}$alkylthio, trifluoromethyl, hydroxy, nitro, amino and mono- and di-($C_{1-3}$alkyl)amino; or phenyl fused with a non-aromatic, heterocyclic ring having 5- or 6-ring members including 1 or 2 hetero atoms selected from the group consisting of oxygen and/or sulphur,
with the proviso that when $R_2''$ is hydrogen, neither $R_3''$ nor $R_4''$ is methyl,
in free form or in pharmaceutically acceptable salt form.